# EUROPÄISCHE PATENTANMELDUNG

(11) **EP 2 765 404 A1**
(43) Veröffentlichungstag der Anmeldung: **13.08.2014**
(21) Anmeldenummer: 13191928.4
(22) Anmeldetag: 07.11.2013
(51) Int. Cl.: G01N 5/04, G01N 15/06, G01N 33/28

(54) **Verfahren und Vorrichtung zur Analyse von Flüssigkeiten**

(30) Priorität: 09.02.2013 CH 4212013
(71) Anmelder: Cofac AG, 4614 Hagendorf (CH)
(72) Erfinder: Zingg, Andreas, 4616 Hägendorf (CH)
(74) Vertreter: Spierenburg, Pieter

(57) **Zusammenfassung**

Es wird ein neues Verfahren zur Analyse von Flüssigkeiten beschrieben, wobei die zu analysierende Flüssigkeit mittels Unterdruck in ein Messgefäss (2) hineingesaugt wird, dessen Verschluss (3) mit einer Filtermembran (9) versehen ist, um die Filtrierbarkeit der Flüssigkeit zu erfassen, und das Gewicht des Messgefässes (2), welches sich durch die eingesaugte Flüssigkeit stetig verändert, in regelmässigen Zeitabständen bei einer definierten Temperatur mittels einer Waage (4) gemessen wird. Die erhaltenen Messwerte (Ist-Werte) werden mit den Messwerten der nicht-verunreinigten Flüssigkeit (Soll-Werte) verglichen. Bei einer Abweichung ausserhalb eines vorbestimmten Schwellenwertes werden die Resultate automatisch gekennzeichnet, welche dem Benutzer Informationen über die Flüssigkeit geben, um diese weiter zu beobachten und/oder weitere Massnahmen zu ergreifen.

## Beschreibung

Die Erfindung betrifft ein Verfahren und eine Vorrichtung zur Analyse von Flüssigkeiten.

### STAND DER TECHNIK

Für die eingesetzte Technik - wie Pumpen, Ventile, Lager, Zahnräder, Düsen usw. - in Hydraulik- und Schmierölsystemen, thermischen Anlagen, Bearbeitungsmaschinen und Reinigungsanlagen ist eine stetige Zustandskontrolle der eingesetzten Flüssigkeit, wie Hydraulik- und Schmieröle, Bearbeitungsöle, Kühlschmierstoffe, Reiniger und Kraftstoffe, für einen sicheren Betrieb von eminenter Bedeutung. Verunreinigungen in flüssigkeitsbetriebenen Systemen führen zu Verschleiss und anderen Problemen welche zu einem zunehmenden Ausfallrisiko führen.

Durch Flüssigkeitsanalysen und andere systembezogene Messwerte werden Rückschlüsse über den Anlagenzustand bezüglich Verschleiss, Kühlerleckagen, Flüssigkeitsalterung und dergleichen gezogen und Massnahmen getroffen, wie beispielsweise Flüssigkeitsreinigung oder Ersetzen von defekten Komponenten oder Austausch der Flüssigkeit. Dazu werden Flüssigkeitsproben im Labor auf ihre Eigenschaften und Zustände hin, analysiert.

Flüssigkeitsproben müssen korrekt entnommen, sicher verpackt und meistens in ein Labor transportiert werden. Die Abwicklung ist aufwendig und mit Wartezeiten verbunden. Je nach Flüssigkeit, Gebinde und Verpackung besteht auf dem Transportweg auch ein Risiko einer Zustandsänderung der Flüssigkeitsprobe. Obwohl die Analyse im Labor korrekt durchgeführt wird, kann dies zu einem nicht mehr repräsentativen Resultat führen und somit zu einer Fehlbeurteilung.

Eingeschränkt durch Anwendung, Flüssigkeitsart und Flüssigkeitszustand werden einzelne Messverfahren, wie beispielsweise mit einem Partikelzähler oder Wassersensor, auch in Flüssigkeitssystemen vor Ort eingesetzt. Solche Messverfahren sind in der Handhabung oft aufwendig und mit Investitionen verbunden, welche nur in einem stark eingeschränkten Anwendungsbereich genutzt werden können, so dass in vielen Fällen eine Laboranalyse vorgezogen wird.

### AUFGABE DER ERFINDUNG

Der vorliegenden Erfindung liegt die Aufgabe zugrunde, ein Analyseverfahren anzugeben, welches unabhängig von Flüssigkeitsart und Flüssigkeitszustand unmittelbar ein leicht nachvollziehbares Gesamtbild über den Flüssigkeitszustand ergibt, woraus sofort Rückschlüsse über den Zustand des Systems (Anlage, Aggregat, Maschine) gezogen werden können. Eine weitere Aufgabe der Erfindung ist es, eine geeignete Vorrichtung zur Analyse von Flüssigkeiten anzugeben.

### GEGENSTAND DER ERFINDUNG

Diese Aufgabe wird durch ein Verfahren zur Analyse von Flüssigkeiten mit den Merkmalen des Patentanspruchs 1 und durch eine Vorrichtung zur Analyse von Flüssigkeiten mit den Merkmalen des Patentanspruchs 5 gelöst.

### VORTEILE DER ERFINDUNG

Mit der Erfindung wird das Filtrationsverhalten der zu prüfenden Flüssigkeit aufgezeichnet und mit Referenzwerten der gleichen Flüssigkeit bei einem festgelegten Zustand automatisch verglichen. Es können sofort Rückschlüsse über die Verunreinigungen in fester, flüssiger und gasförmiger Art gezogen und ein detaillierter Bericht der Analyse generiert werden.

Die Erfindung hat den Vorteil, dass ein Analyseverfahren für unterschiedliche Flüssigkeitssysteme, zur Feststellung von Veränderungen, eingesetzt werden kann und das Resultat sofort im Vergleich zu der systembezogenen Referenzprobe vorliegt. Das erfindungsgemässe Verfahren kann sowohl an einem System vor Ort als auch in einem Labor angewendet werden.

Das Verfahren eignet sich besonders gut für Hydraulik- und Schmieröle, Getriebeund Motorenöle, Turbinenöle, Bearbeitungsöle, Lebensmittelöle, Transformatorenöle, Wärmeträgeröle, wasserbasierende Kühlschmierstoffe (Emulsionen, Lösungen), industrielle Reiniger, Kältemittel und andere vakuumtaugliche Flüssigkeiten.

Das Verfahren dient zur Zustandskontrolle von flüssigkeitsbetriebenen Systemen (Anlagen, Aggregate und Maschinen) im Vergleich zu Referenzkurven.

Das Analyseverfahren ergibt ein Gesamtbild über die Feststoff-Verunreinigung (Rückstände auf der Filtermembran), die flüssige Verunreinigung (Veränderung der Filtrierbarkeit), die gasförmige Verunreinigung (Veränderung der Schaumbildung unter definiertem Vakuum) und Veränderungen ölspezifischer Eigenschaften, welche sich in der Filtrierbarkeit auswirken.

### BESCHREIBUNG EINES AUSFÜHRUNGSBEISPIELES DER ERFINDUNG

Weitere Vorteile der Erfindung folgen aus den abhängigen Patentansprüchen und aus der nachfolgenden Beschreibung, in welcher die Erfindung anhand schematischer Zeichnungen dargestellt und eines Ausführungsbeispiels näher erläutert wird. Es zeigt:
- Fig. 1: ein Analysegerät zur Durchführung des Verfahrens,
- Fig. 2: die Dateneingabe in eine Datenbank, und
- Fig. 3: eine Analyse mit einer Filtrationskurve im Vergleich zu einer Referenz.

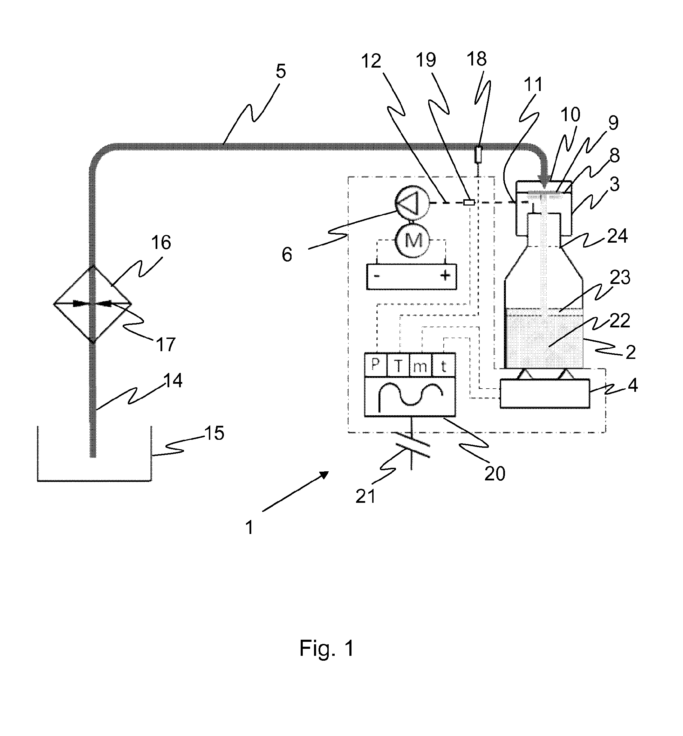

In Figur 1 ist rein schematisch eine Vorrichtung 1 zur Analyse von Flüssigkeiten dargestellt, welches als mobiles Analysegerät ausgebildet ist. Die Vorrichtung 1 besteht aus einem Messgefäss 2, einem Verschluss 3 für das Messgefäss 2, einer elektronischen Präzisionswaage 4, einer Zuleitung 5 und einer elektrischen Vakuumpumpe 6. Im Verschluss 3 ist eine Halterung 8 für eine Filtermembran 9 vorgesehen. Ferner weist der Verschluss 3 eine Kupplung 10 für die Zuleitung 5 und einen Vakuumanschluss 11 für eine Vakuumleitung 12 auf, welche mit der Vakuumpumpe 6 verbunden ist. Das vordere Ende 14 der Zuleitung 5 ist in einem Gefäss 15 mit der zu analysierenden Flüssigkeit eingeführt. Ferner ist im vorderen Bereich der Zuleitung 5 eine Heizung 16 mit einer Temperaturregelung 17 und im Bereich des Verschlusses 3 ist ein Temperatursensor 18 vorgesehen. Damit kann die zu analysierende Flüssigkeit auf eine genau definierte Prüftemperatur gebracht werden. Das Aufheizen und Regulieren der zu analysierenden Flüssigkeit kann auch durch verschiedene andere Methoden erfolgen. Beispielsweise kann die zu analysierende Flüssigkeit direkt unter der Betriebstemperatur aus dem Gefäss 15 (z.B. Flüssigkeitstank einer Maschine) entnommen werden oder das Gefäss 15 in einem (nicht-dargestellten) Wärmeschrank erwärmt werden oder die zu analysierende Flüssigkeit kann durch eine Heizung mit Magnetrührer (nicht dargestellt) im Gefäss 15 erwärmt werden. Andererseits oder zusätzlich könnte die Zuleitung 5 über eine grössere Länge mittels eines (nicht-dargestellten) Heizschlauches beheizbar sein, womit die zu analysierende Flüssigkeit auf die gewünschte Prüftemperatur erwärmt wird bzw. erhalten bleibt. Ein Unterdrucksensor 19 ist in der Vakuumleitung 12 vorgesehen, um den Unterdruck im Messgefäss 2 zu überwachen. Der Unterdruck wird entsprechend der maximalen Pumpenleistung der Vakuumpumpe 6 aufgebaut. Wenn nötig kann über ein Ventil (nicht dargestellt) in der Vakuumleitung 12 ein beliebig reduzierter Unterdruck exakt eingestellt werden. Ein Messcomputer 20 ist mit der Präzisionswaage 4, mit dem Temperatursensor 18 und mit dem Unterdrucksensor 19 elektrisch verbunden. Am Messcomputer 20 ist ferner eine Datenschnittstelle 21 vorgesehen, um die Daten auf einen (hier nicht gezeigten) mobilen Datenspeicher, beispielsweise einen USB Stick oder SD-Karte, zu laden.

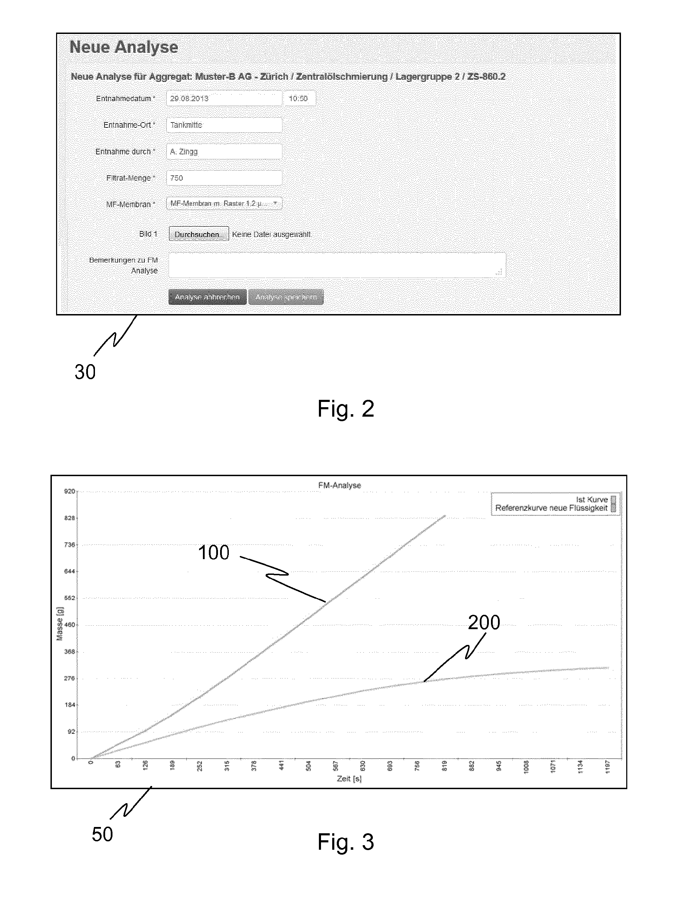

In Figur 2 ist eine Eingabemaske 30 für die Analyse dargestellt. Die vom Messcomputer 20 aufgenommen Daten werden mittels einer nicht-dargestellten Datenübermittlung zu einer externen Datenbank in einem Server übermittelt und können über die Eingabemaske 30 zur Auswertung gelangen.

Figur 3 zeigt eine Grafik 50 aus einem Bericht, wie das Analyseresultat oder die gemessenen Ist-Kurve 200 mit der hinterlegte Referenzkurve 100 grafisch verglichen wird. Dabei ist auf der Abszisse die Zeit in Sekunden aufgetragen und auf der Ordinate die Masse des Filtrats in Gramm.

Die Funktion der Vorrichtung 1 ist nun folgendermassen: durch den im Messgefäss 2 erzeugten Unterdruck wird die zu messenden Flüssigkeit über die Filtermembran 9 in das Messgefäss 2 gezogen. Auf der Filtermembran 9 bleiben die Rückstände in der Flüssigkeit zurück und das Filtrat 22 fliesst in das Messgefäss 2. Das Gewicht des einströmenden Filtrats 22 wird in regelmässigen Zeitabständen bei einer definierten Temperatur von der elektronischen Präzisionswaage 4 gemessen und die Messdaten werden vom Messcomputer 20 erfasst und gespeichert. Je nach Verunreinigung der zu messenden Flüssigkeit, welche von fester, flüssiger oder gasförmiger Art sein kann, ergeben die Messdaten einen unterschiedlichen Verlauf der Ist-Kurve 200 (Fig. 3). Bei fester oder flüssiger Verunreinigung führt dies zu einer zunehmenden Verblockung der Filtermembran 9, so dass der Durchfluss durch die Filtermembran 9 stetig verringert wird. Dies kann sogar zu einer vollständigen Verblockung der Filtermembran 9 führen, so dass kaum noch Flüssigkeit durch die Filtermembran 9 fliesst. Bei gasförmiger Verunreinigung, wie beispielsweise in Öl gelöster Luft, führt dies zu einer zunehmenden Schaumbildung 23 oberhalb des Filtrats 22. Dadurch wird die Filtrat-Menge geringer ausfallen, was im Bericht automatisch angegeben wird. Die erhaltenen Messwerte (Ist-Werte) werden mit den Messwerten der nicht-verunreinigten Flüssigkeit (Soll-Werte) verglichen. Bei einer Abweichung ausserhalb eines vorbestimmten Schwellenwertes werden die Resultate automatisch gekennzeichnet, welche dem Benutzer Informationen über die Flüssigkeit geben, um diese weiter zu beobachten und/oder weitere Massnahmen zu ergreifen.

Wenn das maximale Gewicht des eingezogenen Filtrats im Messgefäss 2 erreicht wird, stellt die Vakuumpumpe 6 automatisch ab und beendet die Datenaufzeichnung vom Messcomputer 20. Dadurch ist gewährleistet, dass eine saubere und automatisierte Messung der Flüssigkeit durchgeführt werden kann. Der Kontrollaufwand durch die Bedienungsperson wird dadurch erheblich verringert.

In einer Datenbank, welche beispielsweise auf einem Server hinterlegt ist und mittels einer gesicherten Verbindung über eine Homepage zugänglich ist, können die Referenzdaten für die zu messenden Flüssigkeiten hinterlegt sein. Der Kunde kann sich nun mit seinen Zugangsdaten auf der Homepage einloggen, das verwendete Flüssigkeitssystem aus vorgegebenen Angaben auswählen und die Messdaten über eine Internet-Verbindung einlesen. Aus den Messdaten wird durch ein Auswerte-Programm eine Ist-Kurve 200 erstellt. Um Veränderungen festzustellen, wird die Referenzkurve 100 in der Grafik 50 ebenfalls dargestellt. Diese Grafik 50 bildet Teil eines automatisch generierten Berichts, in welchem die Filtrat-Menge, die Filtrationszeit, die Flüssigkeitstemperatur, den Unterdruck und die Daten der Filtermembran 9 dokumentiert werden.

Bei Bedarf kann die Filtermembran 9 fotografiert und das Bild ergänzend zu den Messdaten in der Datenbank aufgenommen und im Bericht automatisch integriert werden oder mittels eines Mikroskops untersucht oder für weiterführende Analysen verwendet werden. So können beispielsweise detaillierte Rückschlüsse über die Verunreinigungsart (z.B. glänzende, nicht-glänzende Partikel, Fasern, Partikelgrössen) gezogen und gezielte Massnahmen getroffen werden.

## Patentansprüche

1. Verfahren zur Analyse von Flüssigkeiten, wobei die zu analysierende Flüssigkeit mittels Unterdruck in ein Messgefäss (2) hineingesaugt wird, dessen Verschluss (3) mit einer Filtermembran (9) versehen ist, um die Filtrierbarkeit der Flüssigkeit zu erfassen, und das Gewicht des Messgefässes (2), welches sich durch die eingesaugte Flüssigkeit stetig verändert, in regelmässigen Zeitabständen bei einer definierten Temperatur mittels einer Waage (4) gemessen wird, und die erhaltenen Messwerte (Ist-Werte) mit den Messwerten der nicht-verunreinigten Flüssigkeit (Soll-Werte) verglichen werden, und bei einer Abweichung ausserhalb eines vorbestimmten Schwellenwertes die Flüssigkeit weiter beobachtet wird und/oder weitere Massnahmen ergriffen werden.

2. Verfahren nach Anspruch 1, **dadurch gekennzeichnet, dass** die zu analysierende Flüssigkeit auf eine vorbestimmte Prüftemperatur erwärmt wird.

3. Verfahren nach Anspruch 1 oder 2, **dadurch gekennzeichnet, dass** die Messwerte automatisch in einen Computer eingegeben werden, um eine Messwert-Kurve zu erstellen.

4. Verfahren nach Anspruch 3, **dadurch gekennzeichnet, dass** die Messwerte kontinuierlich erfasst werden.

5. Vorrichtung zur Durchführung des Verfahrens nach einem der Ansprüche 1 bis 4, welche ein Messgefäss (2), dessen Verschluss (3) mit einer Filtermembran (9) versehen ist, eine Waage (4) zur Messung des Gewichts des Messgefässes (2), eine Vakuumpumpe (6), welche über einer Vakuumleistung (12) an einem Anschluss (11) des Messgefässes (2) angeschlossen ist, und ein Computer (20) zur Erfassung und Auswertung der Messwerte aufweist.

6. Vorrichtung nach Anspruch 5, **dadurch gekennzeichnet, dass** eine Zuleitung (5) von einem Gefäss (15) mit der zu analysierenden Flüssigkeit zum Verschluss (3) des Messgefässes (2) führt.

7. Vorrichtung nach Anspruch 6, **dadurch gekennzeichnet, dass** die Zuleitung (5) von einem Heizschlauch beheizbar ist.

8. Vorrichtung nach Anspruch 6 oder 7, **dadurch gekennzeichnet, dass** das Gefäss (15) in einem Wärmeschrank vorgesehen ist, um die zu analysierende Flüssigkeit auf eine vorbestimmte Prüftemperatur zu bringen.

9. Vorrichtung nach Anspruch 6 oder 7, **dadurch gekennzeichnet, dass** das Gefäss (15) eine Heizung mit Magnetrührer aufweist, um die zu analysierende Flüssigkeit auf eine vorbestimmte Prüftemperatur zu bringen.
